# EUROPÄISCHE PATENTSCHRIFT

(11) **EP 1 478 666 B1**
(45) Veröffentlichungstag und Bekanntmachung des Hinweises auf die Patenterteilung: **25.05.2011**
(21) Anmeldenummer: 02802267.1
(22) Anmeldetag: 30.10.2002
(51) Int. Cl.: C07K 14/78, C07K 5/068, A61K 38/39, A61K 8/64, A61P 17/00, A61K 38/05

(54) **DERMOPHARMAZEUTISCH UND KOSMETISCH WIRKSAME OLIGOPEPTIDE**
DERMOPHARMACEUTICALLY AND COSMETICALLY ACTIVE OLIGOPEPTIDES
OLIGOPEPTIDE A ACTION DERMOPHARMACEUTIQUE ET COSMETIQUE

(30) Priorität: 30.10.2001 CH 198601
(43) Veröffentlichungstag der Anmeldung: 24.11.2004
(73) Patentinhaber: DSM IP Assets B.V., 6411 TE Heerlen (NL)
(72) Erfinder: LUDIN, Christian, CH-4147 Aesch (CH); HEIDL, Marc, 79639 Grenzach-Wyhlen (DE); ZIEGLER, Hugo, CH-4108 Witterswil (CH)
(74) Vertreter: Braun, André jr.
(86) Internationale Anmeldenummer: PCT/CH2002/000587
(87) Internationale Veröffentlichungsnummer: WO 2003/037933

(56) Entgegenhaltungen:
- WO-A-00/15188
- WO-A-00/62740
- WO-A-86/04334
- KOU KATAYAMA: "A PENTAPEPTIDE FROM TYPE I PROCOLLAGEN PROMOTES EXTRACELLULAR MATRIX PRODUCTION*" JOURNAL OF BIOLOGICAL CHEMISTRY, AMERICAN SOCIETY OF BIOLOGICAL CHEMISTS, BALTIMORE, MD, US, Bd. 268, Nr. 14, 15. Mai 1993 (1993-05-15), Seiten 9941-9944, XP000367509 ISSN: 0021-9258 in der Anmeldung erwähnt

## Beschreibung

Die vorliegende Erfindung betrifft pharmazeutisch bzw. dermopharmazeutisch und kosmetisch wirksame oligopeptide, deren Derivate, Peptid Mimetika und deren Derivate sowie deren Verwendung in dermopharmazeutisch und kosmetisch aktiven Zusammensetzungen.

Es ist bekannt, dass sich der grundlegende Mechanismus der Hautalterung in der extrazellulären Matrix abspielt, welche als Basallamelle bezeichnet wird, wenn sie sich an der Schnittstelle des Epithels und des Bindegewebes befindet. Die Wiederherstellung der extrazellulären Matrix ist von Bedeutung, da diese das Verhalten der Zellen, die mit ihr in Kontakt stehen, insbesondere deren Entwicklung, Migration, Wucherung, Form und Funktionen wesentlich beeinflusst. Alters bedingt findet in dieser extrazellulären Matrix eine Verringerung der Synthese von Kollagen durch die Fibroblasten statt, was eine Verringerung der Anzahl von diesen Zellen abgesonderten chemischen Stoffe zur Folge hat. Da die Hautproteine zu etwa 80% aus Kollagen bestehen, kann naturgemäss bereits eine kleine Verringerung der Kollagenkonzentration im Gewebe deutliche Folgen für die mechanischen und physiologischen Eigenschaften der Haut haben.

Katayama et al. (The Journal of Biological Chemistry, Vol. 268, Nr. 14, Seiten 9941-9944, 1993) fanden, dass die minimale Sequenz des Subfragments zur Stimulation von Collagen und Fibronectin durch das Pentapeptid Lys-Thr-Thr-Lys-Ser dargestellt wird. Sequenzen beinhaltend vier Aminosäuren oder weniger haben eine geringere oder keine stimulatorische Wirkung.

In der europäischen Patentanmeldung WO 00/15188 wird die Wirkung des palmitoylierten Pentapeptids Palm-Lys-Thr-Thr-Lys-Ser als Inhaltsstoff zur Behandlung von Hautalterung, zur Beschleunigung der Wundheilung und zur Verbesserung der Hydratisierung der Haut beschrieben.

Es wurde nun gefunden, dass ausgewählte neue Oligopeptide und deren Derivate, sowie die Peptid Mimetika und deren Derivate (im weiteren als "erfindungsgemässe Verbindungen" bezeichnet) überraschend stark pharmazeutisch und/oder kosmetisch wirksam und insbesondere für die Verwendung in dermopharmazeutisch und/oder kosmetisch wirksamen Zusammensetzungen geeignet sind. Die erfindungsgemässen Verbindungen diffundieren schnell und in ausreichender Konzentration durch die Zellmembran zum intrazellulären Wirkort und bewirken dort eine deutlich erhöhte Produktion von Collagen und Fibronectin. Somit haben die erfindungsgemässen Verbindungen einen überraschend positiven und stimulierenden Einfluss auf die extracelluläre Matrix, welche das mechanische und physiologische Aussehen der Haut entscheidend beeinflusst. Insbesondere bewirken die erfindungsgemässen Verbindungen eine sehr viel schnellere und stärkere Stimulierung der Collagensynthese als dies bislang aus dem Stand der Technik für andere Verbindungen bekannt war. Vermutlich ist die überraschend schnellere und stärkere Stimulierung der Collagensynthese der erfindungsgemässen Verbindungen einer synergistischen Wirkung zu zuschreiben, welche durch die Reduktion des Molekulargewichts, durch die Einführung von N-Methylgruppen an Aminosäuren sowie durch die Modifikation der Aminosäuren mit z.B. Fettsäureresten, erhalten wird. Die vorliegende Erfindung ist aber nicht an diese Erklärung gebunden.

Die vorliegende Erfindung ist in den Patentansprüchen definiert und betrifft insbesondere ausgewählte neue Oligopeptide und deren Derivate, Peptid Mimetika und deren Derivate, sowie pharmazeutisch akzeptable Salze dieser Verbindungen, dadurch gekennzeichnet, dass diese Verbindungen der allgemeinen Formel (I) 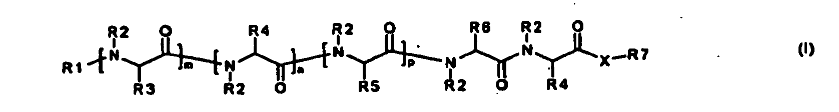 entsprechen, worin
R¹ = H, -C(O)-R7, -S02-R7, -C(O)-OR7 oder -C(O)-N(R7)2,
R² = unabhängig voneinander H oder -(C₁-C₄)-Alkyl,
R³ = -(CH₂)_{q}-N(R¹)R⁸,
R⁴ =, R⁵ = unabhängig von einander -CH₂-OR², -CH(CH₃)OR⁸ oder -CH₂-CH₂-OR⁸,
R⁶ = -(CH₂)ᵣ-N(R¹)R⁸,
R⁷ Wasserstoff, gegebenenfalls ein- oder mehrmals unabhängig von einander mit Halogen, Hydroxy, Carboxyl, Amino, Merkapto, 1,2-Dithiolanyl, und/oder Sulfo substituiertes (C₁-C₁₉)-Alkyl: gegebenenfalls substituiertes C₁₇-C₁₉-Alkenyl; Phenyl-C₁-C₄-alkyl, dessen Phenylrest gegebenenfalls in p-Stellung mit Amino substituiert ist; oder
R⁸ = H, -(C₁-C₄)-Alkyl, -C(O)-R⁷, -C(O)-OR⁷, -C(O)-N(R7)₂ oder -SO₂-R⁷;
X = Sauerstoff (-0-) oder -NH-; oder XR⁷ = mit X = 0 auch die Ester von α-Tocopherol, Tocotrienol oder Retinol, oder die Carbonsäure (mit R⁷ = H), m, n, p = unabhängig voneinander Null oder 1, ihre Summe aber 1, 2, oder 3, q = in R³ eine ganze Zahl von 1 bis 4, und r in R⁶ eine ganze Zahl von 1 bis 3 bedeuten.

Vorzugsweise bedeuten nicht gleichzeitig R⁴ -CH₂-OH oder -CH(CH₃)-OH und R⁵ = -CH(CH₃)-OH. Vorzugsweise bedeuten nicht gleichzeitig R² = Hund R⁴ = -CH₂-OH oder -CH(CH₃)-OH und R⁵= -CH(CH₃)-OH.

Die Verbindungen der Formel (I) umfasst Tripeptide, Tetrapeptide und Pentapeptide der entsprechenden Aminosäuren und deren Derivate entsprechend den Definitionen von R¹, R², R⁷ und R⁸.

Die Reste der Aminosäuren in der allgemeinen Formel (I), welche die Substituenten R³ oder R⁶ enthalten, sind von Ornithin (Orn), 2,4-Diaminobuttersäure (Dab) oder von 2, 3-Diaminopropionsäure (Dap), im Fall von R³ auch von Lysin (Lys), abgeleitet und sind Reste dieser Aminosäuren oder dieser Aminosäurederivate.

Die Reste der Aminosäuren in der allgemeinen Formel (I), welche die Substituenten R⁴ oder R⁵ enthalten, sind von Serin (Ser), Threonin (Thr), Homoserin [H₂N-CH((CH₂)₂-OH)COOH,(HSe)], abgeleitet und sind Reste dieser Aminosäuren oder dieser Aminosäurederivate.

Tabellarisch zusammengestellt, sind die Verbindungen der obigen Formel (I) von den folgenden Aminosäuren abgeleitet:

| R¹(H(R²)CH(R³)C(O) Abgeleitet von: | N(R²)CH(R⁴)C(O) abgeleitet von: | N(R²)CH(R⁵)C(O) abgeleitet von: | N(R²)CH(R⁶)C(O) abgeleitet von: | N(R²)CH(R⁴)C(X)R⁷ abgeleitet von: |
|---|---|---|---|---|
| Lysin (Lys) Ornthin (Orn) 2,4-Diaminobuttersäure (Dab) 2,3-Diaminopropionsäure (Dap) | Serin (Ser) Homoserin(HSe) Threonin (Thr) | Serin (Ser) Homoserin(HSe) Threonin (Thr) | Ornithin (Orn) 2,4-Diaminobuttersäure (Dab) 2,3-Diaminopropionsäure (Dap) | Serin (Ser) Homoserin (HSe) Threonin (Thr) |

Der Rest [R¹(N(R²)CH(R³)C(O)-] ist vorzugsweise abgeleitet von Lysin.

Der Rest [-N(R²)CH(R⁴) C(O)-] an der Stelle [-N(R²)CH(R⁴)C(O)-N(R²)CH(R⁵)C(O)-] ist vorzugsweise abgeleitet von Threonin.

Der Rest [-N(R²)CH(R⁵)(O)-] ist vorzugsweise abgeleitet von Threonin.

Der Rest [-N(R²)CH(R⁶)C(O)-] ist abgeleitet von Ornithin, 2,4-Diaminobuttersäure (Dab), und 2,3-Diaminopropionsaure (Dap).

Der endständige Rest [-(N(R²)CH(R⁴)C(O)-XR⁷] ist vorzugsweise abgeleitet von Serin.

Bevorzugt enthalten die Verbindungen der Formel (I) die folgenden Sequenzen:
Sequenz 1: -Ser-Ser-Orn-
Sequenz 2: -Thr-Thr-Orn-
Sequenz 3: -Thr-Thr-Dab-
Sequenz 4: -Thr-Thr-Dap-

Insbesondere bevorzugt sind Verbindungen mit den Sequenzen -Ser-Ser-Orn-, Lys-Thr-Thr-Orn-Ser, Lys-Thr-Thr-Dab-Ser und Lys-Thr-Thr-Dab-Ser, sowie die entsprechend mit den Substituenten R¹, R², R⁷ und R⁸ derivatisierten Sequenzen. Weitere bevorzugte Polypetide, welche unter die Verbindungen der Formel (I) fallen, sind im Text angegeben.

Unter dem Begriff "Peptide" und "Oligopeptide° sind insbesondere natürlich vorkommende Aminosäuren bzw. Peptide und Oligopeptide zu verstehen. Im Rahmen der vorliegenden Erfindung bedeuten Derivate insbesondere Aminosäuren bzw. Peptide und Oligopeptide, deren terminale Aminogruppe oder Carboxylgruppe weiter umgesetzt wurde, z.B. worin die terminale Carboxylgruppe verestert wurde.

Die Verbindungen der Formel (I) sind insbesondere geeignet als pharmazeutische bzw. dermopharmazeutische und/oder kosmetische Wirkstoffe für die Herstellung von pharmazeutisch bzw. dermopharmazeutisch und/oder kosmetisch wirksamen Zusammensetzungen, insbesondere für die Erhöhung der Produktion von Collagen und Fibronectin in der menschlichen Haut.

Die vorliegende Erfindung betrifft im weiteren Verfahren zur Herstellung der erfindungsgemässen Verbindungen und deren Salze und deren Verwendung als pharmazeutische bzw. dermopharmazeutische und/oder kosmetische Wirkstoffe, sowie pharmazeutisch bzw. dermopharmazeutisch und/oder kosmetisch wirksame Zusammensetzungen, welche mindestens eine erfindungsgemässe Verbindung enthalten.

Die vorliegende Erfindung betrifft im weiteren die Verwendung der erfindungsgemässen Verbindungen für die Zubereitung eines Medikaments zur Förderung der Wundheilung und der Hydratation, sowie Verfahren zur Verzögerung oder Behandlung der Hautalterung, insbesondere der Faltenbildung, welches dadurch gekennzeichnet ist, dass man eine erfindungsgemässe Verbindung und/oder eine erfindungsgemässe Zusammensetzung auf die Haut appliziert.

Die oben verwendeten, allgemeinen Ausdrücke sind wie folgt definiert:

Halogen bedeutet Chlor, Brom oder Jod, vorzugsweise Fluor. Unter Alkyl, als Gruppe per se oder als Strukturelement einer Alkoxyfunktion, sind sowohl lineare wie verzweigte Alkylgruppen zu verstehen. Beispiele sind Methyl, Ethyl, Propyl, n-Butyl, n-Pentyl, n-Hexyl, n-Heptyl, n-Octyl, n-Nonyl, n-Undecanyl, n-Dodecanyl, n-Tridecanyl, n-Hexadecanyl, n-Heptadecanyl, n-Octadecanyl oder n-Nonadecanyl als unverzweigte Reste und Isopropyl, tert.Butyl, Isobutyl, sec.Butyl, Isoamyl als verzweigte Reste. R² und/oder **R⁸** als Alkyl bedeutet vorzugsweise Methyl, Ethyl und Propyl, vorzugsweise Methyl. Bedeutet oder enthält R⁷ als Teil von R¹ einen Alkylrest, so bedeutet dieser vorzugsweise Alkyl mit 8 bis 22 C-Atomen, vorzugsweise mit 14 bis 17 C-Atomen. Enthält XR⁷ einen Alkylrest, so bedeutet dieser vorzugsweise Alkyl mit 1 bis 22 C-Atomen, vorzugsweise mit 1 bis 4 C-Atomen.

Alkenyl hat die Bedeutung einer ein- oder mehrfach ungesättigten, gegebenenfalls substituierten Alkylgruppe, wie z.B. 8(Z)-Heptadecenyl, 8(Z),11(Z)-Heptadecadienyl, 4(Z),7(Z),10(Z),13(Z)-Nonadecatetraenyl, 8(Z)-11-Hydroxy-octadecenyl.

α-Tocopheryl bedeutet (D)-, (L)- oder (DL)-2,5,7,8-Tetramethyl-2-(d',e',12'-trimethyltridecyl)-6-chromanyl. Tocotrienyl bedeutet ein beliebiges Isomeres von 2,5,7,8-Tetramethyl-2-(4',8',12'-trimethyl-3',7',11'-tridecatrienyl)-6-chromanyl. Retinyl bedeutet 3,7-Dimethyl-9-(2,6,6-trimethyl-1-cyclohexenyl)-2,4,6,8-nonatetraen-1-yl.

Die Verbindungen der Formel (I) können mit Säuren ein- oder mehrwertige, einheitliche oder gemischte Salze bilden, z.B. mit anorganischen Säuren, wie Salzsäure, Bromwasserstoffsäure, Schwefelsäure oder Phosphorsäure; oder mit geeigneten organischen alifatischen gesättigten oder ungesättigten Carbonsäuren, z.B. aliphatischen Mono- oder Dicarbonsäuren, wie Ameisensäure, Essigsäure, Trifluoressigsäure, Trichloressigsäure, Propionsäure, Glykolsäure, Bernsteinsäure, Fumarsäure, Malonsäure, Maleinsäure, Oxalsäure, Phthalsäure, Zitronensäure, Milchsäure oder Weinsäure; oder mit aromatischen Carbonsäuren, wie Benzoesäure oder Salicylsäure; oder mit aromatisch-aliphatischen Carbonsäuren, wie Mandelsäure oder Zimtsäure; oder mit heteroaromatischen Carbonsäuren, wie Nikotinsäure; oder mit aliphatischen oder aromatischen Sulfonsäuren, wie Methansulfonsäure oder Toluolsulfonsäure. Bevorzugt sind dermatologisch verträgliche Salze, insbesondere Salze mit Essigsäure und/oder Milchsäure.

Die Verbindungen der allgemeinen Formel (I) umfassen auch die möglichen isomeren Formen sowie deren Gemische, z.B. racemische Gemische und Gemische von Rotameren.

Die in Formel (I) angeführten Aminosäuren können die L-Form, die D-Form oder ein Gemisch aus beiden Formen darstellen. Insbesondere Thr kann auch die isomeren Formen allo-Thr, D-Thr oder D-allo-Thr bzw. eine Mischung von Thr mit D-Thr bzw. allo-Thr mit D-allo-Thr an der jeweiligen Stelle in der Sequenz bedeuten.

Von den Verbindungen der Formel (I) sind die folgenden Bedeutungen bzw. die folgende Gruppen von Verbindungen bevorzugt. Diese Gruppen sind jene, worin
R¹ = Wasserstoff, -C(O)-R⁷-SO₂-R⁷-C(O)-OR⁷ oder -C(O)-N(R⁷)₂, vorzugsweise worin R¹ = Wasserstoff, -C(O)-R⁷ oder -SO₂-R⁷, vorzugsweise Wasserstoff oder -C(O)-R⁷, R² = unabhängig von einander Wasserstoff oder Methyl, R³=(CH₂)_{q}-N(R¹)R⁸, vorzugsweise -(CH₂)_{q}-NH₂, R⁴, R⁵ = unabhängig von einander -CH₂-OR⁸, -CH(CH₃)OR⁸ oder -CH₂-CH₂-OR⁸, vorzugsweise unabhängig von einander -CH₂-OH, -CH(CH₃)OH oder -CH₂-CH₂-OH,
R⁶ =-(CH₂)ᵣ-N(R¹)R⁸, vorzugsweise -(CH₂)r-NH₂
R⁷ = vorzugsweise Wasserstoff, Methyl, Ethyl, Propyl, n-Butyl, n-Pentyl, n-Hexyl, n-Heptyl, n-Octyl, n-Nonyl, n-Undecanyl, n-Dodecanyl, n-Tridecanyl, n-Tetradecanyl, n-Pentadecanyl n-Hexadecanyl, n-Heptadecanyl, n-Octadecanyl oder n-Nonadecanyl, Isopropyl, tert.Butyl, Isobutyl, sec.Butyl, Isoamyl, Phenyl, t-Butylphenyl, Tolyl, 1- oder 2-Naphthyl, Perfluorbutyl, Pentadecafluoroheptyl, (+)- oder (-)-Bornan2-onyl,8(Z)-Heptadecenyl, 8(Z),11(Z)-Heptadecadienyl, 4(Z),7(Z),10(Z),13(Z)-Nonadecatetraenyl oder 8(Z)-11-Hydroxyoctadecenyl, 1,2-Dithiolanyl oder in ω-Stellung mit Amino substituiertes C₁-C₁₉-Alkyl, oder gegebenenfalls in ortho- und/oder para-Stellung mit Methyl, Amino, oder Halogen substituiertes Phenyl, vorzugsweise R⁷ als Teil von XR⁷ Wasserstoff, Methyl, Ethyl und/oder R⁷ als Teil von R1 einen Alkylrest mit 14 bis 17 C-Atomen.
R⁸ = Wasserstoff, C₁-C₄-Alkyl oder -C(O)-R⁷, vorzugsweise Wasserstoff oder Methyl.
X = Sauerstoff, -NH-, vorzugsweise Sauerstoff,
XR⁷= mit X = 0 auch die Ester von α-Tocopherol, Tocotrienol oder Retinol, oder die Carbonsäure (mit R⁷ = H), vorzugsweise die Carbonsäure bedeuten.

Besonders bevorzugt bedeutet R⁷-C(O)- den Rest einer gesättigten oder ungesättigten Fettsäure mit 6, 8, 10, 12, 14, 16 oder 18 C-Atomen, vorzugsweise den entsprechenden Rest einer gesättigten Fettsäure, vorzugsweise den entsprechenden Rest der Caprylsäure [CH₃-(CH₂)₆-C(O)-], Laurinsäure [CH₃-(CH₂)₁₀-C(O)-], Myristinsäure [CH₃-(CH₂)₁₂-C(O)-], Palmitinsäure [CH₃-(CH₂)₁₄-C(O)-] und/oder Stearinsäure [CH₃-(CH₂)₁₆-C(O)-].

Repräsentative Beispiele von Verbindungen der Formel (I) sind in Tabelle 5 (nach Beispiel 7) aufgelistet.

Die erfindungsgemässen Verbindungen können unter Verwendung von an sich in der Peptidchemie bekannten Methoden hergestellt werden. Vorzugsweise geht man so vor, dass man die erfindungsgemässe Verbindung, z.B. eine Verbindung der Formel (I) vollständig aufbaut, gegebenenfalls die verbleibende(n) Schutzgruppe(n) abspaltet und gegebenenfalls eine freie Aminogruppe acyliert und/oder die erhaltene Verbindung in ein Säureadditionssalz und/oder ein erhaltenes Säureadditionssalz in die entsprechende konjugate Base oder in ein anderes Salz überführt.

Die erfindungsgemässen Verbindungen können zur Herstellung einer dermopharmazeutisch und/oder kosmetisch wirksamen Zusammensetzung verwendet werden. Solche Zusammensetzungen enthalten eine wirksame Menge mindestens einer erfindungsgemässen Verbindung oder eines Salzes davon, im Bereich zwischen 0,5 ppm und 5.000 ppm (w/w), vorzugsweise zwischen 1 ppm und 1000 ppm (w/w), berechnet auf das Gewicht der erfindungsgemässen Verbindung und des Trägermaterials bzw. der Trägermaterialien. Die erfindungsgemässen Verbindungen können in Form einer Lösung, einer Dispersion, einer Emulsion oder eingekapselt in Trägern, wie z.B. in Makro-, Mikro- oder Nanokapseln, in Liposomen oder Chylomikronen, oder eingeschlossen in Makro-, Mikro- oder Nanoteilchen oder in Mikroschwämme oder absorbiert auf pulverförmigen organischen Polymeren, Talk, Bentonit und anderen mineralischen Trägern, verwendet werden.

Die erfindungsgemässen Verbindungen können in jeder galenischen Form verwendet werden wie beispielsweise: Emulsionen, Milch, Lotionen, Salben, gelierende und viskose, spannungsaktive und emulgierende Polymere, Pommaden, Shampoos, Seifen, Gele, Puder, Sticks und Stifte, Sprays, Körperöle, Gesichtsmasken oder eines Pflasters zur transdermalen Applikation.

Die erfindungsgemässen Verbindungen können mit jedem anderen üblicherweise verwendeten Inhaltsstoff verwendet werden, wie beispielsweise: Extraktionslipide und/oder Syntheselipide, gelierende und viskose, spannungsaktive und emulgierende Polymere, wasser- oder fettlösliche Wirkprinzipien, Pflanzenextrakte, Gewebeextrakte, Meeresextrakte, Sonnenschutzmittel, Antioxidantien, Feuchthalte- und Barrieremittel, Haut revitalisierende Wirkstoffe.

Die erfindungsgemässen Verbindungen werden in den kosmetischen Anwendungen eingesetzt, um die die Hydratation zu begünstigen, und für alle Hautpflegemittel, insbesondere gegen die Bildung und die Verschlimmerung der Falten und gegen alle Folgen der Hautalterung natürlicher oder beschleunigter Art (Heliodermie, Verschmutzung).

Die erfindungsgemässen Verbindungen sowie die kosmetischen und dermopharmazeutischen Verbindungen, die sie enthalten, können für die Zubereitung eines Medikaments zur Förderung der Wundheilung, der Hydratation und für alle Hautpflegeprodukte eingesetzt, insbesondere gegen die Bildung und die Verschlimmerung der Falten und gegen alle Folgen der Hautalterung natürlicher oder beschleunigter Art (Heliodermie, Verschmutzung) verwendet werden.

In diesem Sinne betrifft die vorliegende Erfindung auch ein Verfahren zur Verzögerung oder Behandlung der Hautalterung, insbesondere der Faltenbildung, indem man eine erfindungsgemässe Verbindung auf die Haut appliziert. Analogerweise betrifft die vorliegende Erfindung ein Verfahren zur Verbesserung der Hydratisierung der Haut, welches dadurch gekennzeichnet ist, dass man eine erfindungsgemässe Verbindung auf die Haut appliziert.

Die folgenden Beispiele erläutern die Erfindung ohne sie einzuschränken. Die folgenden Abkürzungen werden im Text und in den Beispielen 1-7 benutzt:
- AcOH:: Essigsäure
- Boc:: tert.-Butyloxycarbonyl
- Dab:: 2,4-Diaminobuttersäure
- Dap:: 2,3-Diaminopropionsäure
- DBU:: 1,8-Diazabicyclo[5,4,0]undec-7-ene (1,5-5)
- DIPEA:: Diisopropylethylamin
- DMEM:: Dulbecco's Modified Eagle Medium
- Et:: Ethyl
- FCS:: Foetal Calf Serum
- HSe:: Homoserin
- Hyp:: Hydroxyprolin
- Gly:: Glycin
- Lipoyl:: α-D,L-Liponsäure
- Me:: Methyl
- MEM:: Minimal Essential Medium
- N-Me-Ser:: N-Methyl-Serin
- N-Me-Thr:: N-Methyl-Threonin
- NMM:: N-Methylmorpholin
- Orn:: Ornithin
- Palm:: Palmitoyl
- PBS:: Phosphate buffered saline
- Pr:: Propyl
- RT:: Raumtemperatur
- TBTU:: O-(Benzotriazol-1-yl)-N,N,N',N'- tetramethyluronium-tetrafluoroborat
- tBu:: t-Butyl
- TFA:: Trifluoressigsäure
- TGF-β(beta)1:: Tranforming Growth Factor-beta(β)

### Beispiel 1: Bestimmung der Stimulierung der Collagensynthese Typ I+III in Fibroblasten-Zellkulturen (ATCC CCL110) durch Behandlung mit den erfindungsgemässen Oligopeptidderivaten.

### Methode:

Die Messung von Collagen I und III Gehalt in Fibroblasten wurde mit dem Sirius Red Mikroassay durchgeführt. Die quantitative Bestimmung von Kollagen erfolgte nach 24 Stunden Inkubationszeit mit den entsprechenden Oligopeptidderivaten. Es wurde das extrazellulär angereicherte Kollagen bestimmt. Als Positivkontrollen dienen Vitamin C und TGF-beta-1.

### Methode:

Fibroblasten ATCC CCL110 wurden mit einer Dichte von ca. 125000 Zellen/Well in 6-Well Zellkulturplatten für zwei Tage in Kulturmedium inkubiert (37°C/5%CO₂). Danach wird das Medium verworfen und 2mal mit PBS gewaschen und 1ml Testmedium mit entsprechenden Testsubstanzen zugegeben. Die Inkubation mit Testsubstanz erfolgt unter Kulturbedingungen (37°C/5%CO₂) für weitere 24 Stunden. Danach wird mit dem Sirius Red Assay das extrazelluläre Kollagen bestimmt.

### Sirius Red Assay extrazellulär:

Nach der Inkubationszeit wird das gesamte Volumen des Testmediums (1ml) in eine "flat-bottom" 24-well Platte transferiert und über Nacht bei 55°C getrocknet. Die Proben werden fixiert mit 1 ml/Well Bouin's fluid für 1 Stunde bei Raumtemperatur (RT). Die Fixationslösung wird entfernt und die Platten mit Wasser 2-3 Mal gewaschen. Die Platten werden getrocknet bevor 1 ml Sirius Red dye Reagenz hinzugefügt wird.

Die Proben werden 1 Stunde bei RT auf einem Mikroplattenschüttler bei mittlerer Intensität geschüttelt. Danach wird das Sirius Red dye Reagenz entfernt und die Platten mit 0.01N HCl gewaschen. Das gefärbte Material wird in 0.3 ml 0.1N NaOH Lösung gelöst und auf einem Mikroplattenschüttler geschüttelt 1 Stunde bei Raumtemperatur.

200 µl Lösung werden zu einem "flat-bottom 96-well plate" transferiert und die Optische Dichte bei 540 nm gegen 0.1N NaOH Lösung als Blank gemessen.

### Material:

Kulturmedium: MEM + 10% FCS + 100IU/ml Penicillin + 0.1mg/ml Streptomycin + 1 mM nicht essentielle Aminosäuren + 1 mM Na-Pyruvat + 2 mM L-Glutamin.

Testmedium: DMEM ohne Phenolrot (AMIMED) + 100IU/ml Penicillin + 0.1mg/ml Streptomycin + 80µg/ml beta(β)-Aminopropionitril.

### Substanzverdünnung:

Vitamin C 100 mM (SIGMA A4034) wird verdünnt auf 50 µg/ml im Testmedium.

Transforming growth factor TGFβ1 1µg/ml (SIGMA T1654) wird verdünnt auf 0.1 ng/ml in Testmedium.

Die erfindungsgemässen Oligopeptidderivate werden in einer Stammkonzentration von 10 mM hergestellt und auf 50 µM im Testmedium verdünnt.

### Reagentien für den Sirius Red Mikroassay:

Sirius Red F3BA dye reagent als Stammlösung 1mg/ml in gesättigter wässriger Pikrinsäurelösung. Bouin's fluid: 15. ml gesättigte wässrige Pikrinsäurelösung + 5 ml Formaldehyd + 1ml Essigsäure. Resultate sind in Tabelle 1 aufgelistet.

**Tabelle 1**

| Resultate, Mengenbestimmung Kollagen Typ I+III nach 24Std.: | |
|---|---|
| Testsubstanz, wenn nicht anders vermerkt bei einer Konzentration von 50 µm | Extrazellulär |
| Kontrolle ohne wirkstoff | 100% |
| TGFβ1 0.1 ng/ml | 115% |
| Vitamin C | 128% |

### Beispiel 2: Bestimmung des Fibronectin Gehalts nach der Behandlung mit den erfindungsgemässen Oligopeptidderivaten Methode:

Die Bestimmung des Fibronectin(Laminin oder KollagenVII)-Gehaltes der Fibroblastenzellen efolgt über die Immuno-Dot-Blot Methode. Die Kultivierung und Inkubation der Zellen erfolgt nach dem gleichen Verfahren wie in Beispiel 1. Die Zellen werden danach lysiert und die Lysate (0.5ml) in einer vorbereiteten Bio-Dot Apparatur auf eine Nitrocellulose-membran geblottet. Die Membranen wurden anschliessend nach dem Western-Blot Verfahren mit einem spezifischen primären Antikörper gegen humanes Fibronectin inkubiert. Ein Konjugat mit alkalischer Phosphatase diente als sekundärer Antikörper. Die Bandenintensität wurde nach dem Färben und Abstoppen der Reaktion von Auge halbquantitativ bestimmt. Die Resultate sind in Tabelle 2 aufgelistet.

**Tabelle 2**

| Resultate, Mengenbestimmung von Fibronectin (oder Laminin V oder Kollagen Typ VII) nach 24 Stunden Inkubation: | |
|---|---|
| Testsubstanz, wenn nicht anders vermerkt bei einer Konzentration von 50 µM | Extrazellulär (relative Färbungsintensität) |
| Kontrolle ohne Wirkstoff | ++ |
| TGFβ1 0.1 ng/ml | ++ |
| Vitamin C | +++(+) |
| H-Lys-Thr-Thr-DaB-Ser-OH x 3 AcOH | ++++ |

### Beispiel 3: Formulierung einer Salbe

Prozedur: Die Inhaltstoffe 1-5 (A) werden auf 70°C erhitzt. Die Inhaltstoffe 6-7 (B) werden auf 75°C erhitzt. Unter Rühren wird B zu A gegeben, auf 50°C gekühlt, homogenisiert und auf 30°C abgekühlt. Dann werden die Inhaltstoffe 8-9 (C) und der Inhaltsstoff 10 (D) nacheinander hinzu gegeben und kalt gerührt. Die Formulierungen sind in Tabelle 3 wieder gegeben.

**Tabelle 3**

| Nummer | Inhaltsstoff | % w/w |
|---|---|---|
| 1 | (A) Tego Care 450 | 3.00 |
| 2 | Cetearylalkohol | 2.25 |
| 3 | Gycerylstearat | 2.25 |
| 4 | Cetiol 868 | 10.00 |
| 5 | Squalane | 5.00 |
| 6 | (B) Deionisiertes Wasser | 66.995 |
| 7 | Natriumhyaluronat | 5.00 |
| 8 | (C) Glycerin | 5.00 |
| 9 | Phenonip | 0.5 |
| 10 | (D) Oktyl-1-SO₂-**Lys-Seg-Ser-Dab-Ser-OH** | 0.005 |

### Beispiel 4: Formulierung eines Gels (zur Illustration

Prozedur: Die Inhaltstoffe 2-6 (A) werden nacheinander in deionisiertem Wasser gelöst. Mit Inhaltstoff 7 (B) wird auf pH 6.0 gestellt. Dann wird Inhaltstoff 8 (C) hinzugegeben. Die Formulierungen sind in Tabelle 4 wieder gegeben.

**Tabelle 4**

| Nummer | Inhaltsstoff | % w/w |
|---|---|---|
| 1 | (A) Deionisiertes Wasser | 92.095 |
| 2 | 1,3-Butandiol | 5.00 |
| 3 | Phenonip | 0.50 |
| 4 | Abil B 8843 | 1.50 |
| 5 | Carboxymethyl Cellulose | 0.15 |
| 6 | Carbopol Ultrez 10 | 0.75 |
| 7 | (B) NaOH | |
| 8 | (C) Oktyl-SO₂-Lys-Ser-Ser-Lys-Ser-OH | 0.005 |

### Beispiele 5-6:

In den nachfolgenden Ausführungsbeispielen 5 und 6 wird die Herstellung von erfindungsgemässen Verbindungen der Formel (I) und von Salzen solcher Verbindungen beschrieben. Die Analyse der gemäss den Beispielen erhaltenen Eluate und Produkte wurde mit Proton-NMR, HPLC-Elektrospray-MS oder Elementaranalyse ausgeführt. Die Verbindungen können nach den im folgenden beschriebenen an sich bekannten Verfahren (allgemeinen Vorschriften von M. Bodanszky "The Practice of Peptide Synthesis" Springer Verlag, 2nd Edition 1994) hergestellt werden. Entsprechend wird die Aminosäure, beispielsweise Serin, in einer Festphasen Synthese am carboxyterminalen Ende an ein Harz angeknüpft, wobei deren Aminogruppe durch eine Schutzgruppe, z.B. durch die Fmoc-Schutzgruppe, geschützt wird. Die Seitenkette wird z.B. mit Boc oder t-Butyl geschützt. Die Schutzgruppen werden nach Bedarf selektiv abgespalten, um die weiteren Aminosäure Derivate mit den in der Peptid Synthese üblichen Reagentien anzuknüpfen bis die gewünschte Kettenlänge vollständig aufgebaut ist. Das Peptid resp. Peptid Mimetikum wird dann am carboxyterminalen Ende vom Harz abgespalten und dieses carboxylterminale Ende mit unterschiedlichen C(O)-R⁷, SO₂-R⁷, C(O)-OR⁷ oder C(O)-N(R⁷)₂ -reste verknüpft. Die Schutzgruppen werden anschliessend entfernt.

### Beispiel 5: H-Lys-Thr-Ser-Orn-Ser x 3 TFA

### Herstellung von H-Lys(Boc)-Thr(tBu)-Ser(tBu)-Orn(Boc)-Ser(tBu)-OH x TFA (5a):

In einem typischen Festphasen Synthese Protokoll wurden durch repetitive Kopplung von 18.7 g (14.0 mmol, Beladung: 0.75 mmol/g) käuflichem H-Ser(tBu)-chlorotritylharz mit 16.8 mmol der Aminosäuren Fmoc-Orn(Boc)-OH, Fmoc-Thr(tBu)-OH (2x), Fmoc-Lys (Boc)-OH 18 mmol TBTU, 33.6 mmol Collidin und Deblockierung mit 1% DBU in DMF (2 x 5 min) das Pentapeptid aufgebaut, mit 1% TFA in Dichlormethan vom Harz abgespalten und über Sephadex LH20® gereinigt, Ausbeute: 9.3 g (64%).

Herstellung von H-Lys-Thr-Ser-Orn-Ser-OH x 3TFA (5b) 9.3 g 5a wird in einer Mischung aus 59 ml TFA, 1.25 ml Wasser und 1.25 ml Triisopropylsilan bei RT 1 h gerührt. Nach Einengen auf 1/3 des Volumens wird mit Diethylether ausgefällt, Ausbeute: 6.0 g (76%)

Beispiel 6: [Herstellung von CH₃-(CH₂)₇-SO₂-Lys-Thr-Ser-Orn-Ser x 2 TFA (6]): 2 g (2.2 mmol) 5a wird in DMF (20 ml) gelöst und mit 0.48 g (2.3 mmol) 1-Oktansulfochlorid und 0.48 g (4.0 mmol) DIPEA bei RT 10 h gerührt. Nach Abdestillieren des Lösungsmittels wird das Rohprodukt mit 59 ml TFA, 1.25 ml Wasser und 1.25 ml Triisopropylsilan bei RT 1 h gerührt. Nach Einengen auf 1/3 des Volumens wird mit Diethylether ausgefällt und über Sephadex LH20® gereinigt, Ausbeute: 1.4 g (70%)

Das Peptid oder Konjugat kann auch mit einer Mineralsäure, z.B. HCl, HBr, H₂SO₄ oder H₃PO₄, oder mit einer organischen Säure, z.B. Ameisensäure, Oxalsäure oder Weinsäure, protoniert werden, wobei man entsprechende Salze von 6 erhält.

Nach den in den Beispielen 5 und 6 beschriebenen Methoden lassen sich auch folgende Verbindungen herstellen:

**Tabelle 5:**

| Nr. | Sequenz | ESI-MS |
|---|---|---|
| 1 | Palm-Lys-Thr-Thr-Dap-Ser-OH x 2AcOH | 760.8 |
| 2 | Palm-Lys-Thr-Thr-Dab-Ser-OH x 2AcOH | 775.0 |
| 3 | Laurinoyl-Lys-Thr-Thr-Orn-Ser-OH x 2AcOH | 732.8 |
| 4 | Myristinoyl-Lys-Thr-Thr-Orn-Ser-OH x 2AcOH | 760.8 |
| 5 | Stearinoyl-Lys-Thr-Thr-Orn-Ser-OH x 2AcOH | 817.1 |
| 6 | Palmitoleinoyl-Lys-Thr-Thr-Orn-Ser-OH x 2AcOH | 787.1 |
| 7 | Oleoyl-Lys-Thr-Thr-Orn-Ser-OH x 2AcOH | 815.0 |
| 8 | Eicosaenoyl-Lys-Thr-Thr-Orn-Ser-OH x 2AcOH | 843.1 |
| 9 | Palm-Lys-Thr-Thr-Orn-Thr-OH x 2AcOH | 803.1 |
| 10 | Palm-Lys-Ser-Ser-Orn-Ser-OH x 2AcOH | 761.0 |
| 11 | Octadecyl-NH-C(O)-Lys-Ser-Ser-Orn-Ser-OH x 2AcOH | 846.1 |
| 12 | Hexadecyl-NH-C(O)-Lys-Ser-Ser-Orn-Ser-OH x 2AcOH | 818.1 |
| 13 | Tetradecyl-NH-C(O)-Lys-Ser-Ser-Orn-Ser-OH x 2AcOH | 790.1 |
| 14 | Palm-Lys-Thr-Thr-Orn-Ser-OH x 2AcOH | 788.9 |
| 15 | Palm-Lys-Thr-Thr-Orn-N-Me-Ser-OH x 2AcOH. | |
| 16 | Palm-Lys-Thr-Thr-N-Me-Orn-Ser-OH x 2AcOH | |
| 17 | Palm-Lys-Thr-N-Me-Thr-Orn-Ser-OH x 2AcOH | |
| 18 | Palm-Lys-N-Me-Thr-Thr-Orn-Ser-OH x 2AcOH | |
| 19 | Palm-N-Me-Lys-Thr-Thr-Orn-Ser-OH x 2AcOH | |
| 20 | Palm-Lys-N-Et-Thr-Thr-Orn-Ser-OH x 2AcOH | |
| 21 | Palm-Lys-N-Pr-Thr-Thr-Orn-Ser-OH x 2AcOH | |
| 22 | C₉H₁₇-SO₂-Lys-Thr-Thr-Orn-Ser-OH x 2AcOH | 726.8 |
| 23 | C₁₆H₃₃-SO₂-Lys-Thr-Thr-Orn-Ser-OH x 2AcOH | 839.1 |
| 24 | C₇F₁₅-C(O)-Lys-Thr-Thr-Orn-Ser-OH x 2AcOH | 946.8 |
| 25 | H-Lys-Thr-Thr-Orn-Ser-ORetinyl 3 x 2AcOH | |

## Patentansprüche

1. Oligopeptide und deren Derivate, Peptid Mimetika und deren Derivate, sowie pharmazeutisch akzeptable Salze dieser Verbindungen, **dadurch gekennzeichnet, dass** diese Verbindungen der allgemeinen Formel (I) 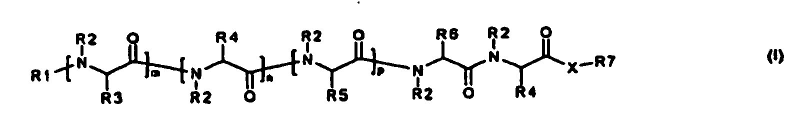 entsprechen, worin ,
R¹ = H, -C(O)-R⁷ -SO₂-R⁷, -C(O)-OR⁷ oder -C(O)-N (R⁷)₂
R² = unabhängig von einander H oder -(C₁-C₄)-Alkyl,
R³ = -(CH₂)₄-N (R¹)R⁸,
R⁴, R⁸ = unabhängig von einander -CH₂-OR², -CH(CH₃)OR⁸ oder -CH₂-CH₂-OR⁸,
R⁶ = -(CH₂)ᵣ-N(R¹)R⁸,
R⁷ = Wasserstoff, gegebenenfalls ein- oder mehrmals unabhängig von einander mit Halogen, Hydroxy, Carboxyl, Amino, Merkapto, 1,2-Dithiolanyl, und/oder Sulfo substituiertes (C₁-C₁₉) -Alkyl; gegebenenfalls substituiertes C₁-C₁₉-Alkenyl; Phenyl-C₁-C₄-alkyl, dessen Phenylrest gegebenenfalls in p-Stellung mit Amino substituiert ist; oder
R⁸ = H, -(C₁-C₄)-Alkyl, -C(O)-R⁷, -C(O)-OR⁷, -C(O)-N(R⁷)₂ oder -SO₂-R⁷
X = Sauerstoff (-O-) oder -NH-; oder
XR⁷ = mit X = O auch die Ester von α-Tocopherol, Tocotrienol oder Retinol, oder die Carbonsäure (mit R⁷ = H),
m, n, p = unabhängig voneinander Null oder 1, ihre Summe aber 1, 2 oder 3, q = in R³ eine ganze Zahl von 1 bis 4 und
r = in R⁶ eine ganze Zahl von 1 bis 3 bedeuten.

2. Verbindungen nach Anspruch 1, **dadurch gekennzeichnet,**
**dass** nicht gleichzeitig R4 = -CH₂-OH oder -CH(CH₃)-OH und R⁵= -CH(CH₃)-OH bedeuten.

3. Verbindungen nach Anspruch 2, **dadurch gekennzeichnet, dass** nicht gleichzeitig R² = H und R⁴ = -CH₂-OH oder - CH(CH₃)-OH und R⁵ = -CH(CH₃)-OH bedeuten.

4. Verbindungen nach einem der Ansprüche 1-3, **dadurch gekennzeichnet, dass** der Rest [R¹(N(R²)CH(R³)C(O)-] von Lysin abgeleitet ist.

5. Verbindungen nach einem der Ansprüche 1-4, **dadurch gekennzeichnet, dass** der Rest [-N(R²)CH(R⁴)C(O)-] an der Stelle [-N(R²)CH(R⁴)C(O)-N(R²)CH(R⁵)C(O)-] von Threonin abgeleitet ist.

6. Verbindungen nach einem der Ansprüche 1-5, **dadurch gekennzeichnet, dass** der Rest [-N(R²)CH(R⁵)C(O)-] von Threonin abgeleitet ist.

7. Verbindungen nach einem der Ansprüche 1-6, **dadurch gekennzeichnet, dass** der endständige Rest [-(N(R²)CH(R⁴)C(O)-XR⁷] von Serin abgeleitet ist.

8. Verbindungen nach einem der Ansprüche 1-7, **dadurch gekennzeichnet, dass** diese eine der folgenden Sequenzen enthält:
Sequenz 1: -Ser-Ser-Orn-
Sequenz 2: -Thr-Thr-Orn-
Sequenz 3: -Thr-Thr-Dab-
Sequenz 4: -Thr-Thr-Dap-

9. Verbindungen nach einem der Ansprüche 1-8, **dadurch gekennzeichnet, dass** diese eine der folgenden Sequenzen: - Ser-Ser-Orn-, Lys-Thr-Thr-Orn-Ser, Lys-Thr-Thr-Dab-Ser oder Lys-Thr-Thr-Dab-Ser, sowie die entsprechend mit den Substituenten R¹, R², R⁷ und R³ derivatisierten Sequenzen, enthält.

10. Verbindungen nach einem der Ansprüche 1-9, **dadurch gekennzeichnet, dass** Alkyl in R² und/oder **R⁸** unabhängig voneinander Methyl, Ethyl und Propyl, vorzugsweise Methyl; und in R¹ und/oder R⁷ Alkyl mit 1 bis 4 oder 8 bis 22 C-Atomen, vorzugsweise mit 1 oder 2 bzw. 14 bis 17 C-Atomen, bedeuten.

11. Verbindungen nach einem der Ansprüche 1-10, **dadurch gekennzeichnet, dass** die Verbindungen der Formel (I) mit Säuren ein- oder mehrwertige, einheitliche oder gemischte Salze bilden, vorzugsweise mit anorganischen Säuren, oder mit geeigneten organischen alifatischen gesättigten oder ungesättigten Carbonsäuren, oder mit aromatischen Carbonsäuren, oder mit aromatisch-aliphatischen Carbonsäuren, oder mit heteroaromatischen Carbonsäuren, oder mit aliphatischen oder aromatischen Sulfonsäuren, vorzugsweise mit Essigsäure und/oder Milchsäure.

12. Verbindungen nach einem der Ansprüche 1-11, **dadurch gekennzeichnet, dass** sie als isomeren Formen und deren Gemische, und als Gemische von Rotameren, vorliegen.

13. Verbindungen nach einem der Ansprüche 1-12, **dadurch gekennzeichnet, dass**
R¹ = Wasserstoff, -C(O)-R⁷ oder -SO₂-R⁷, vorzugsweise -C(O)-R⁷ oder Wasserstoff
R² = unabhängig von einander Wasserstoff oder Methyl,
R³-(CH₂)_{q}-NH₂,
R⁴ und R⁵ unabhängig von einander -CH₂-OH, -CH(CH₃)OH oder CH₂-CH₂-OHO,
R⁶-(CH₂)ᵣ-NH₂
R⁷ Wasserstoff, Methyl, Ethyl, Propyl, n-Butyl, n-Pentyl, n-Hexyl, n-Heptyl, n-Octyl, n-Nonyl, n-Undecanyl, n-Dodecanyl, n-Tridecanyl, n-Tetradecanyl, n-Pentadecanyl n-Hexadecanyl, n-Heptadecanyl, n-Octadecanyl, n-Nonadecanyl, Isopropyl, tert.Butyl, Isobutyl, sec.Butyl, Isoamyl, Phenyl, t-Butylphenyl, Tolyl, 1- oder 2-Naphthyl, Perfluorbutyl, Pentadecafluoroheptyl, (+)- oder (-)-Bornan-2-onyl, 8(Z)-Heptadecenyl, 8(Z),11(Z)-Heptadecadienyl,
4(Z),7(Z),10(Z),13(Z)-Nonadecatetraenyl oder 8(Z)-11-Hydroxyoctadecenyl, 1,2-Dithiolanyl oder in w-Stellung mit Amino substituiertes C₁-C₁₉-Alkyl, oder gegebenenfalls in ortho- und/oder para-Stellung mit Methyl, Amino, oder Halogen substituiertes Phenyl, vorzugsweise R⁷ als Teil von XR⁷ Wasserstoff, Methyl, Ethyl und/oder R⁷ als Teil von R¹ einen Alkylrest mit 14 bis 17 C-Atomen,
R⁸ Wasserstoff oder Methyl,
X = Sauerstoff oder -NH-, vorzugsweise Sauerstoff,
XR⁷ - mit X = O die Ester von α-Tocopherol, Tocotrienol, oder Retinol, oder die Carbonsäure (mit R⁷ = H), vorzugsweise die Carbonsäure, bedeuten.

14. Verbindungen nach einem der Ansprüche 1-13, **dadurch gekennzeichnet, dass** R⁷-C(O)- den Rest einer gesättigten oder ungesättigten Fettsäure mit 6, 8, 10, 12, 14, 16 oder 18 C-Atomen, vorzugsweise den entsprechenden Rest einer gesättigten Fettsäure, vorzugsweise den entsprechenden Rest der Caprylsäure, Laurinsäure, Myristinsäure, Palmitinsäure und/oder Stearinsäure bedeutet.

15. Verbindungen der Formel:
Octadecyl-NH-C(O)-Lys-Ser-Ser-Orn-Ser-OH x 2AcOH
Hexadecyl-NH-C(0)-Lys-Ser-Ser-Orn-Ser-OH x 2AcOH
Tetradecyl-NH-C(O)-Lys-Ser-Ser-Orn-Ser-OH x 2AcOH
Palm-Lys-Ser-Ser-Orn-Ser-OH x 2ACH

16. Verbindungen der Formel (I):
Palm-Lys-Thr-Thr-Dap-Ser-OH x 2AcOH
Palm-Lys-Thr-Thr-Dab-Ser-OH x 2AcOH

17. Verbindungen der Formel (I):
Laurinoyl-Lys-Thr-Thr-Orn-Set-OH x 2AcON
Myristinoyl-Lys-Thr-Thr-Orn-Ser-OH x 2AcOH
Stearinoyl-Lys-Thr-Thr-Orn-Ser-OH x 2AcOH
Palmitoleinoyl-Lys-Thr-Thr-Orn-Ser-OH x 2AcOH
Oleoyl-Lys-Thr-Thr-Orn-Ser-OH x 2AcOH
Eicosaencyl-Lys-Thr-Thr-Orn-Ser-OH x 2AcOH
Palm-Lys-Thr-Thr-Orn-Thr-OH x 2AcOH
Palm-Lys-Thr-Thr-Orn-Ser-OH x 2AcOH
Palm-Lys-Thr-Thr-Orn-N-Me-Ser-OH x 2AcOH
Palm-Lys-Thr-Thr-N-Me-Orn-Ser-OH x 2AcOH
Palm-Lys-Thr-N-Me-Thr-Orn-Ser-OH x 2AcOH
Palm-Lys-N-Me-Thr-Thr-Orn-Ser-OH x 2AcOH
Palm-N-Me-Lys-Thr-Thr-Orn-Ser-oH x 2AcOH
Palm-Lys-N-Et-Thr-Thr-Orn-Ser-OH x 2AcOH
Palm-Lys-N-Pr-Thr-Thr-Orn-Ser-OH x 2AcOH
C₆H₁₅-SO₂-Lys-Thr-Thr-Orn-Ser-OH x 2AcOH
C₁₆H₃₃-SO₂-Lys-Thr-Thr-Orn-Ser-OH x 2AcOH
C₈F₁₅-C(O)-Lys-Thr-Thr-Orn-Ser-OH x 2AcOH
H-Lys-Thr-Thr-Orn-Ser-ORetinyl x 3AcOH

18. Verfahren zur Herstellung der Verbindungen nach einem der Ansprüche 1-17, **dadurch gekennzeichnet, dass** man unter Verwendung von an sich in der Peptidchemie bekannten Methoden die Verbindung der Formel (I) vollständig aufbaut, gegebenenfalls die verbleibende(n) Schutzgruppe(n) abspaltet und gegebenenfalls eine freie Aminogruppe acyliert und/oder die erhaltene Verbindung in ein Säureadditionssalz und/oder ein erhaltenes Säureadditionssalz in die entsprechende konjugate Base oder in ein anderes Salz überführt.

19. Verbindungen nach einem der Ansprüche 1-17 zur Verwendungen als pharmazeutische, vorzugsweise dermopharmazeutische und/oder kosmetische Wirkstoffe, insbesondere für die Erhöhung der Produktion von Collagen und Fibronectin in der menschlichen Haut.

20. Verwendung der Verbindungen nach einem der Ansprüche 1-17 zur Herstellung von dermopharmazeutisch und/oder kosmetisch wirksamen Zusammensetzungen, insbesondere zur Beschleunigung der Wundheilung und der Hydratation, als Hautpflegemittel, insbesondere zur Verhinderung der Bildung und der Verschlimmerung der Falten und gegen alle Folgen der Hautalterung natürlicher oder beschleunigter Art (Heliodermie, Verschmutzung).

21. Dermopharmazeutisch und/oder kosmetisch wirksame Zusammensetzung, welche mindestens eine Verbindung nach einem der Ansprüche 1-17 enthält, vorzugsweise in einer Menge im Bereich zwischen 0,5 ppm und 5.000 ppm (w/w), vorzugsweise zwischen 1 ppm und 1000 ppm (w/w), berechnet auf das Gewicht der erfindungsgemässen Verbindung und des Trägermaterials bzw. der Trägermaterialien.

22. Zusammensetzung nach Anspruch 21, **dadurch gekennzeichnet, dass** diese in Form einer Lösung, einer Dispersion, einer Emulsion oder eingekapselt in Trägern, vorzugsweise in Makrokapseln, Mikrokapseln oder Nanokapseln, in Liposomen oder Chylomikronen, oder eingeschlossen in Makro-, Miro- oder Nanoteilchen oder in Mikroschwämme oder absorbiert auf pulverförmigen organischen Polymeren, Talk, Bentonit und anderen mineralischen Trägern, vorlegt.

23. Zusammensetzung nach Anspruch 21, **dadurch gekennzeichnet, dass** diese in Form einer Emulsion, Milch, Lotion, Salbe, eines gelierenden und viskosen, spannungsaktiven und e-mulgierenden Polymeren, einer Pommade, eines Shampoos, einer Seife, eines Gels, Puders, Sticks oder Stifts, Sprays, Körperöls, einer Gesichtsmaske oder eines Pflasters zur transdermalen Applikation, vorliegt.

24. Zusammensetzung nach einem der Ansprüche 21-23, **dadurch gekennzeichnet, dass** diese üblicherweise verwendete Inhaltsstoffe enthält, welche ausgewählt sind aus der Gruppe enthaltend: Extraktionslipide und/oder Syntheselipide, gelierende und viskose, spannungsaktive und emulgierende Polymere, wasser- oder fettlösliche Wirkprinzipien, Pflanzenextrakte, Gewebeextrakte, Meeresextrakte, Sonnenschutzmittel, Antioxidantien, Feuchthalte- und Barrieremittel und/oder Haut revitalisierende Wirkstoffe.

25. Zusammensetzung nach einem der Ansprüche 21-24 als dermopharmazeutisch und/oder kosmetisch wirksames Mittel, insbesondere für die Erhöhung der Produktion von Collagen und Fibronectin in der menschlichen Haut, zur Beschleunigung der Wundheilung und der Hydratation, als Hautpflegemittel, insbesondere zur Verhinderung der Bildung und der Verschlimmerung der Falten und gegen alle Folgen der Hautalterung natürlicher oder beschleunigter Art (Heliodermie, Verschmutzung).

26. Kosmetisches Verfahren zur Erhöhung der Produktion von Collagen und/oder Fibronectin in der menschlichen Haut und/oder zur Beschleunigung der Hydratation und/oder zur Verzögerung oder Behandlung der Hautalterung, **dadurch gekennzeichnet, dass** man eine Verbindung nach einem der Ansprüche 1-17 auf die Haut appliziert.

## Claims

1. Oligopeptides and derivatives thereof, peptide mimetics and derivatives thereof as well as pharmaceutically acceptable salts of these compounds, **characterized in that** these compounds all correspond to the general formula (I) 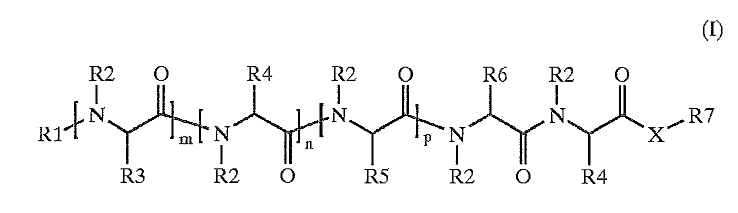 wherein
R¹ = H, -C(O)-R⁷, -SO₂-R⁷, -C(O)-OR⁷ or -C(O)-N(R⁷)₂
R² = independent of one another H or- (C₁-C₄)-alkyl,
R³ = -(CH₂)₄-N(R¹)R⁸,
R⁴, R⁵ = independent of one another -CH₂-OR², -CH(CH₃)OR⁸ or -CH₂-CH₂-OR⁸
R6 = -(CH₂)₁-N(R¹)R⁸
R⁷ = hydrogen, (C₁-C₁₉)-alkyl optionally substituted once or several times independent of one another with halogen, hydroxy, carboxyl, amino, mercapto, 1,2-dithiolanyl, and/or sulfo; optionally substituted (C₁-C₁₉)-alkenyl; phenyl-(C₁-C₄)-alkyl, whose phenyl residue is optionally substituted with amino in the p position; or R⁸ = H, -(C₁-C₄)-alkyl, -C(O)-R⁷, -C(O)-OR⁷, -C(O) - N(R⁷)₂ or - SO₂-R⁷
X = oxygen (-O-) or -NH-; or
XR⁷, where X = O, also represents the esters of α-tocopherol, tocotrienol or retinol or the carboxylic acid (where R⁷ = H),
m, n, p = independent of one another, zero or 1, but the sum thereof represents 1, 2 or 3, q = in R³ an integer from 1 through 4 and r = in R⁶ an integer from 1 through 3.

2. Compounds according to claim 1, **characterized in that** R⁵ is not -CH (CH₃) -OH, if R4 is -CH₂-OH or -CH (CH₃) -OH.

3. Compounds according to claim 2, **characterized in that** R² is not H, if R⁴ is CH₂-OH or -CH (CH₃) -OH and R⁵ is -CH (CH₃) -OH.

4. Compounds according to one of claims 1 - 3, **characterized in that** the residue [R¹(N(R²)CH(R³)C(O)-] is derived from lysine.

5. Compounds according to one of claims 1 - 4, **characterized in that** the residue [-N(R²)CH(R₄)C(O)-] at the position [-N(R²)CH(R⁴)C(O)N(R²)CH(R⁵)C(O) -] is derived from threonine.

6. Compounds according to one of claims 1 - 5, **characterized in that** the residue [-N (R²) CH (R⁵) C (O) -] is derived from threonine.

7. Compounds according to one of claims 1 - 6, **characterized in that** the terminal residue [-(N(R²)CH(R⁴)C(O)-XR⁷] is derived from serine.

8. Compounds according to one of claims 1 - 7, **characterized in that** it contains one of the following sequences:
Sequence 1: -Ser-Ser-Orn-
Sequence 2: -Thr-Thr-Om-
Sequence 3: -Thr-Thr-Dab-
Sequence 4: -Thr-Thr-Dap-

9. Compounds according to one of claims 1 - 8, **characterized in that** it contains one of the following sequences: -Ser-Ser-Orn-, Lys-Thr-Thr-Orn-Ser, Lys-Thr-Thr-Dab-Ser or Lys-Thr-Thr-Dab-Ser, as well as the sequences correspondingly derivatized with the substituents R¹ , R² , R⁷ and R⁸ .

10. Compounds according to one of claims 1 - 9 **characterized in that** alkyl in R² and/or R⁸ represent, independent of one another, methyl, ethyl and propyl, preferably methyl; and in R¹ and/or R⁷ alkyl with 1 to 4 or 8 to 22 C atoms, preferably with 1 or 2, or 14 to 17 C atoms.

11. Compounds according to one of claims 1 - 10, **characterized in that** the compounds of formula (I) together with acids form monovalent or polyvalent, homogeneous or mixed salts, preferably with inorganic acids, or with suitable organic aliphatic saturated or unsaturated carboxylic acids, or with aromatic carboxylic acids, or with aromatic-aliphatic carboxylic acids, or with heteroaromatic carboxylic acids, or with aliphatic or aromatic sulfonic acids, preferably with acetic acid and/or lactic acid.

12. Compounds according to one of claims 1 - 11, **characterized in that** they are present as isomeric forms and mixtures thereof, and as mixtures of rotamers.

13. Compounds according to one of claims 1 - 12, **characterized in that**
R¹ = hydrogen, -C(O)-R⁷ or -SO₂-R⁷, preferably -C(O)-R⁷ or hydrogen
R² = independent of one another, hydrogen or methyl,
R³ = -(CH₂)_{q}-NH₂-
R⁴ and R⁵ represent, independent of one another, -CH₂-OH, -CH(CH₃)OH or - CH₂-CH₂-OH,
R⁶ represents - (CH₂)ᵣ-NH₂
R⁷ represents hydrogen, methyl, ethyl, propyl, n-butyl, n-pentyl, n-hexyl, n-heptyl, n-octyl, n-nonyl, n-undecanyl, n-dodecanyl, n-tridecanyl, n-tetradecanyl, n-pentadecanyl, n-hexadecanyl, n-heptadecanyl, n-octadecanyl, n-nonadecanyl, isopropyl, tert.butyl, isobutyl, sec.butyl, isoamyl, phenyl, t-butylphenyl, tolyl, 1- or 2-naphthyl, perfluorobutyl, pentadecafluoroheptyl, (+)- or (-)-bomane-2-onyl, 8(Z)-heptadecenyl, 8(Z), 11 (Z)-heptadecadienyl, 4(Z),7(Z),10(Z),13(Z)-nonadecatetraenyl or 8(Z)-11-hydroxyoctadecenyl, 1,2-dithiolanyl or (C₁-C₁₉)-alkyl substituted with amino in ω position, or phenyl optionally substituted with methyl, amino or halogen in ortho and/or para position, preferably R⁷ as a part of XR⁷ represents hydrogen, methyl, ethyl and/or R⁷ as a part of R¹ represents an alkyl residue with 14 to 17 C atoms.,
R⁸ represents hydrogen or methyl,
X = oxygen or -NH-, preferably oxygen,
XR⁷ , where X = O, represents the esters of α-tocopherol, tocotrienol or retinol or the carboxylic acid (where R⁷ = H),
preferably the carboxylic acid.

14. Compounds according to one of claims 1 - 13, **characterized in that** R⁷-C(O)-represents the residue of a saturated or unsaturated fatty acid with 6, 8, 10, 12, 14, 16 or 18 C atoms, preferably the corresponding residue of a saturated fatty acid, preferably the corresponding residue of the caprylic acid, lauric acid, myristic acid, palmitic acid and/or stearic acid.

15. Compounds of the formula:
Octadecyl-NH-C(O)-Lys-Ser-Ser-Orn-Ser-OH x 2AcOH,
Hexadecyl-NH-C(O)-Lys-Ser-Ser-Orn-Ser-OH x 2AcOH,
Tetradecyl-NH-C(O)-Lys-Ser-Ser-Orn-Ser-OH x 2AcOH
Palm-Lys-Ser-Ser-Orn-Ser-OH x 2AcOH.

16. Compounds of the formula (I):
Palm-Lys-Thr-Thr-Dap-Ser-OH x 2AcOH,
Palm-Lys-Thr-Thr-Dab-Ser-OH x 2AcOH

17. Compounds of the formula (I):
Laurinoyl-Lys-Thr-Thr-Orn-Ser-OH x 2AcOH,
Myristinoyl-Lys-Thr-Thr-Orn-Ser-OH x 2AcOH,
Stearinoyl-Lys-Thr-Thr-Orn-Ser-OH x 2AcOH,
Palmitoleinoyl-Lys-Thr-Thr-Orn-Ser-OH x 2AcOH,
Oleoyl-Lys-Thr-Thr-Orn-Ser-OH x 2AcOH,
Eicosaenoyl-Lys-Thr-Thr-Orn-Ser-OH x 2AcOH,
Palm-Lys-Thr-Thr-Orn-Thr-OH x 2AcOH,
Palm-Lys-Thr-Thr-Orn-Ser-OH x 2AcOH,
Palm-Lys-Thr-Thr-Orn-N-Me-Ser-OH x 2AcOH,
Palm-Lys-Thr-Thr-N-Me-Orn-Ser-OH x 2AcOH,
Palm-Lys-Thr-N-Me-Thr-Orn-Ser-OH x 2AcOH,
Palm-Lys-N-Me-Thr-Thr-Orn-Ser-OH x 2AcOH,
Palm-N-Me-Lys-Thr-Thr-Orn-Ser-OH x 2AcOH,
Palm-Lys-N-Et-Thr-Thr-Orn-Ser-OH x 2AcOH,
Palm-Lys-N-Pr-Thr-Thr-Orn-Ser-OH x 2AcOH,
C₈H₁₅-SO₂-Lys-Thr-Thr-Orn-Ser-OH x 2AcOH,
C₁₆H₃₃-SO₂-Lys-Thr-Thr-Orn-Ser-OH x 2AcOH,
C₈F₁₅-C(O)-Lys-Thr-Thr-Orn-Ser-OH x 2AcOH,
H-Lys-Thr-Thr-Orn-Ser-ORetinyl x 3AcOH

18. Method for producing the compounds according to one of claims 1 - 17, **characterized in that** the compound of formula (I) is completely built up, using methods known per *se* in peptide chemistry, optionally splitting the remaining protective group(s) and optionally acylating a free amino group and/or converting the obtained compound into an acid addition salt and/or an obtained acid addition salt into the corresponding conjugate base or into another salt.

19. Compounds according to one of claims 1 - 17 for uses as pharmaceutical, preferably dermopharmaceutical and/or cosmetic active substances, in particular for increasing the production of collagen and fibronectin in human skin.

20. Use of the compounds according to one of claims 1 - 17, for producing dermopharmaceutically and/or cosmetically active compositions, in particular to accelerate wound healing and hydration, as skin care products, in particular to prevent the formation and aggravation of wrinkles and against all consequences of natural or accelerated (heliodermia, pollution) skin aging..

21. Dermopharmaceutically and/or cosmetically active composition, which contains at least one compound according to one of claims 1 - 17, preferably in a quantity in the range from 0.5 ppm to 5,000 ppm (w/w), preferably between 1 ppm and 1000 ppm (w/w), calculated on the weight of the compound according to the invention and of the supporting material(s).

22. Composition according to claim 21, **characterized in that** it is present in the form of a solution, a dispersion, an emulsion or encapsulated in carriers, preferably in macrocapsules, microcapsules or nanocapsules, in liposomes or chylomicrons, or enclosed in macroparticles, microparticles or nanoparticles or in microsponges or adsorbed on powdered organic polymers, talc, bentonite and other mineral carriers.

23. Composition according to claim 21, **characterized in that** it is present in the form of an emulsion, a milk, a lotion, an ointment, a gelling and viscous, tensio-active and emulsifying polymer, a pomade, a shampoo, a soap, a gel, a powder, a stick or a bar, a spray, a body oil, a face mask or a plaster for transdermal application.

24. Composition according to one of claims 21 - 23, **characterized in that** it contains commonly used ingredients, which are selected from the group containing:
extraction lipids and/or synthesis lipids, gelling and viscous, surface-active and emulsifying polymers, water-soluble or fat-soluble principles of action, plant extracts, tissue extracts, marine extracts, sunscreen agents, antioxidants, humectants and barrier agents and/or skin-revitalizing active substances.

25. Composition according to one of claims 21 - 24 as a a dermopharmaceutically and/or cosmetically active agent, in particular to increase the production of collagen and fibronectin in human skin, to accelerate wound healing and hydration, as a skin care product, in particular to prevent the formation and aggravation of wrinkles and against all consequences of natural and accelerated (heliodermia, pollution) skin aging.

26. Cosmetic method for increasing the production of collagen and/or fibronectin in human skin and/or to accelerate hydration and/or to delay or treat skin aging, **characterized in that** a compound according to one of claims 1 - 17 is applied to the skin.

## Revendications

1. Oligopeptides et leurs dérivés, mimétiques peptides et leurs dérivés, et sels pharmaceutiquement acceptables de ces composés, **caractérisés par le fait que** ces composés correspondent à la formule générale (I) : 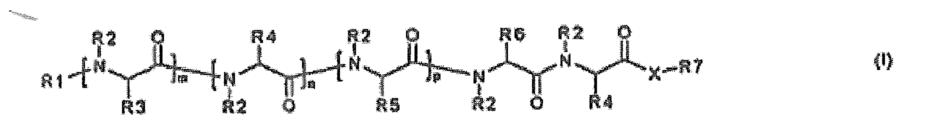 dans laquelle .
R¹ = H, -C(O)-R⁷, SO₂-R⁷, -C(O)-OR⁷ ou -C(O)-N(R⁷)₂ ;
R² = indépendamment les uns des autres, H ou alkyle en C₁-C₄ ;
R³ = (CH₂)₄-N(R¹)R⁸
R⁴, R⁵ = indépendamment l'un de l'autre, -CH₂-OR² -CH(CH₃)OR⁸ ou -CH₂-CH₂-OR⁸ ;
R⁶ = (CH₂)ᵣ-N(R¹)R⁸
R⁷ = hydrogène, alkyle en C₁-C₁₉ éventuellement substitué une ou plusieurs fois indépendamment les unes des autres par halogène, hydroxy, carboxyle, amino, mercapto, 1,2-dithiolanyle et/ou sulfo ; alcényle en C₁-C₁₉ éventuellement substitué ; phényl-alkyle en C₁-C₄, dont le reste phényle est éventuellement substitué en position p par amino ; ou
R⁸ = H, alkyle en C₁-C₄, -C(O)-R⁷, -C(O)-OR⁷, -C(O)-N(R⁷)₂ ou -SO₂-R⁷
X = oxygène (-O-) ou -NH- ; ou
XR⁷ = avec X = O également les esters d'α-tocophérol, tocotriénol ou rétinol ou l'acide carboxylique (avec R⁷ = H),
m, n, p = indépendamment les uns des autres 0 ou 1 mais leur somme valant 1, 2 ou 3, q = dans R³ un nombre total de 1 à 4 et r = dans R⁶ un nombre total de 1 à 3.

2. Composés selon la revendication 1, **caractérisés par le fait que** non simultanément R4 = -CH₂-OH ou -CH(CH₃)-OH et R⁵ = -CH(CH₃)-OH.

3. Composés selon la revendication 2, **caractérisés par le fait que** non simultanément R² = H et R⁴ = -CH₂-OH ou -CH(CH₃)-OH et R⁵ = -CH(CH₃)-OH.

4. Composés selon l'une des revendications 1 à 3, **caractérisés par le fait que** le reste [R¹(N(R²)CH(R³)C(O)-] est dérivé de la lysine.

5. Composés selon l'une des revendications 1 à 4, **caractérisés par le fait que** le reste [-N(R²)CH(R⁴)C(O)-] à la position [-N(R²)CH(R⁴)C(O)-N(R²)CH(R⁵)C(O)-] est dérivé de la thréonine.

6. Composés selon l'une des revendications 1 à 5, **caractérisés par le fait que** le reste [-N(R²)CH(R⁵)C(O)-] est dérivé de la thréonine.

7. Composés selon l'une des revendications 1 à 6, **caractérisés par le fait que** le reste terminal [-N(R²)CH(R⁴)C(O)-XR⁷] est dérivé de la sérine.

8. Composés selon l'une des revendications 1 à 7, **caractérisés par le fait que** ceux-ci contiennent l'une des séquences suivantes :
Séquence 1 : -Ser-Ser-Orn-
Séquence 2 : -Thr-Thr-Orn-
Séquence 3 : -Thr-Thr-Dab-
Séquence 4 : -Thr-Thr-Dap-

9. Composés selon l'une des revendications 1 à 8, **caractérisés par le fait que** ceux-ci contiennent l'une des séquences suivantes : -Ser-Ser-Orn-, Lys-Thr-Thr-Orn-Ser, Lys-Thr-Thr-Dab-Ser ou Lys-Thr-Thr-Dab-Ser ainsi que les séquences transformées en dérivés de façon correspondante avec les substituants R¹, R², R⁷ et R⁸.

10. Composés selon l'une des revendications 1 à 9, **caractérisés par le fait qu'**alkyle dans R² et/ou R⁸ signifient indépendamment les uns des autres méthyle, éthyle et propyle, de préférence méthyle ; et signifient dans R¹ et/ou R⁷, alkyle avec 1 à 4 ou 8 à 22 atomes de C, de préférence avec 1 ou 2 ou 14 à 17 atomes de C.

11. Composés selon l'une des revendications 1 à 10, **caractérisés par le fait que** les composés de formule (I) forment des sels mono- ou plurivalents, homogènes ou mixtes avec les acides, de préférence avec les acides inorganiques ou avec des acides carboxyliques saturés ou insaturés aliphatiques organiques appropriés, ou avec des acides carboxyliques aromatiques, ou avec des acides carboxyliques aromatiques-aliphatiques ou avec des acides carboxyliques hétéroaromatiques ou avec des acides sulfoniques aliphatiques ou aromatiques, de préférence avec de l'acide acétique et/ou de l'acide lactique.

12. Composés selon l'une des revendications 1 à 11, **caractérisés par le fait qu'**il se présente sous des formes isomères et leurs mélanges et comme mélanges de rotamères.

13. Composés selon l'une des revendications 1 à 12, **caractérisés par le fait que**
R¹ = hydrogène, -C(O)-R⁷ ou -SO₂-R⁷, de préférence -C(O)-R⁷ ou hydrogène ;
R² = indépendamment les uns des autres hydrogène ou méthyle ;
R³ = -(CH₂)_{q}-NH₂ ;
R⁴ et R⁵ indépendamment l'un de l'autre = -CH₂-OH, - CH(CH₃)OH ou -CH₂-CH₂-OH ;
R⁶ = -(CH₂)ᵣ-NH₂ ;
R⁷ = hydrogène, méthyle, éthyle, propyle, n-butyle, n-pentyle, n-hexyle, n-heptyle, n-octyle, n-nonyle, n-undécanyle, n-dodécanyle, n-tridécanyle, n-tétradécanyle, n-pentadécanyle, n-hexadécanyle, n-heptadécanyle, n-octadécanyle, n-nonadécanyle, isopropyle, tert.-butyle, isobutyle, sec.-butyle, isoamyle, phényle, t-butylphényle, tolyle, 1- ou 2-naphtyle, perfluorobutyle, pentadécafluoroheptyle, (+)- ou (-)-bornan-2-onyle, 8(Z)-heptadécényle, 8(Z),11(Z)-heptadécadiényle, 4(Z),7(Z),10(Z),13(Z)-nonadécatétraényle ou 8(Z)-11-hydroxyoctadécényle, 1,2-dithiolanyle ou alkyle en C₁-C₁₉ substitué en position w par amino, ou phényle éventuellement substitué en position ortho et/ou para par méthyle, amino ou halogène, de préférence R⁷ comme partie de XR⁷ représente hydrogène, méthyle, éthyle et/ou R⁷ comme partie de R¹ représente un reste alkyle avec 14 à 17 atomes de C ;
R⁸ = hydrogène ou méthyle ;
X = oxygène ou -NH-, de préférence oxygène ;
XR⁷ = avec X = O les esters d'α-tocophérol, tocotriénol ou rétinol ou l'acide carboxylique (avec R⁷ = H), de préférence l'acide carboxylique.

14. Composés selon l'une des revendications 1 à 13, **caractérisés par le fait que** R⁷-C(O)- représente le reste d'un acide gras saturé ou insaturé avec 6, 8, 10, 12, 14, 16 ou 18 atomes de C, de préférence le reste correspondant d'un acide gras saturé, de préférence le reste correspondant de l'acide carprylique, de l'acide laurique, de l'acide myristique, de l'acide palmitique et/ou de l'acide stéarique.

15. Composés de formule :
Octadécyl-NH-C(O)-Lys-Ser-Ser-Orn-Ser-OH x 2AcOH
Hexadécyl-NH-C(O)-Lys-Ser-Ser-Orn-Ser-OH x 2AcOH
Tétradécyl-NH-C(O)-Lys-Ser-Ser-Orn-Ser-OH x 2AcOH
Palm-Lys-Ser-Ser-Orn-Ser-OH x 2AcOH

16. Composés de formule (I) :
Palm-Lys-Thr-Thr-Dap-Ser-OH x 2AcOH
Palm-Lys-Thr-Thr-Dab-Ser-OH x 2AcOH

17. Composés de formule (I) :
Laurinoyl-Lys-Thr-Thr-Orn-Ser-OH x 2AcOH
Myristinoyl-Lys-Thr-Thr-Orn-Ser-OH x 2AcOH
Stearinoyl-Lys-Thr-Thr-Orn-Ser-OH x 2AcOH
Palmitoleinoyl-Lys-Thr-Thr-Orn-Ser-OH x 2AcOH
Oleoyl-Lys-Thr-Thr-Orn-Ser-OH x 2AcOH
Eicosaenoyl-Lys-Thr-Thr-Orn-Ser-OH x 2AcOH
Palm-Lys-Thr-Thr-Orn-Thr-OH x 2AcOH
Palm-Lys-Thr-Thr-Orn-Ser-OH x 2AcOH
Palm-Lys-Thr-Thr-Orn-N-Me-Ser-OH x 2AcOH
Palm-Lys-Thr-Thr-N-Me-Orn-Ser-ON x 2AcOH
Palm-Lys-Thr-Thr-N-Me-Thr-Orn-Ser-ON x 2AcOH
Palm-Lys-N-Me-Thr-Thr-Orn-Ser-ON x 2AcOH
Palm-N-Me-Lys-Thr-Thr-Orn-Ser-OH x 2AcOR
Palm-Lys-N-Et-Thr-Thr-Orn-Ser-OH x 2AcOH
Palm-Lys-N-Pr-Thr-Thr-Orn-Ser-ON x 2AcOH
C₈H₁₅-SO₂-Lys-Thr-Thr-Orn-Ser-OH x 2AcOH
C₁₆H₃₃-SO₂-Lys-Thr-Thr-Orn-Ser-OH x 2AcOH
C₈H₁₅-C(O)-Lys-Thr-Thr-Orn-Ser-OH x 2AcOH
H-Lys-Thr-Thr-Orn-Ser-ORetinyl x 3AcOH

18. Procédé pour la fabrication des composés selon l'une des revendications 1 à 17, **caractérisé par le fait que** l'on forme complètement le composé de formule (I) à l'aide des méthodes connues dans la chimie des peptides, le cas échéant on enlève le ou les groupes protecteurs restants et le cas échéant on acyle un groupe amino libre et/ou on transforme le composé obtenu en un sel d'addition d'acide et/ou on transforme un sel d'addition d'acide obtenu en la base conjuguée correspondante ou en un autre sel.

19. Composés selon l'une des revendications 1 à 17 pour des utilisations comme agents pharmaceutiques, de préférence dermopharmaceutiques et/ou cosmétiques, en particulier pour l'augmentation de la production de collagène et de fibronectine dans la peau humaine.

20. Utilisation des composés selon l'une des revendications 1 à 17 pour la fabrication de compositions dermopharmaceutiquement et/ou cosmétiquement actives, en particulier pour l'accélération de la cicatrisation et de l'hydratation, comme agent de soins cutanés, en particulier pour empêcher la formation et l'aggravation des rides et contre toutes conséquences du vieillissement de la peau de type naturel ou accéléré (héliodermie, pollution).

21. Composition thermopharmaceutiquement et/ou cosmétiquement active, laquelle contient au moins un composé selon l'une des revendications 1 à 17, de préférence dans une quantité dans la plage entre 0,5 ppm et 5 000 ppm (p/p), de préférence entre 1 ppm et 1 000 ppm (p/p), calculée sur la base du poids du composé selon l'invention et du matériau support ou des matériaux supports.

22. Composition selon la revendication 21 **caractérisée par le fait que** celle-ci se présente sous forme d'une solution, d'une dispersion, d'une émulsion ou encapsulée dans des supports, de préférence dans des macrocapsules, des microcapsules ou des nanocapsules, dans des liposomes ou des chylomicrones, ou enfermée dans des macro-, micro- ou nanoparticules ou dans des microéponges ou absorbée sur des polymères organiques pulvérulents, du talc, de la bentonite et autres supports minéraux.

23. Composition selon la revendication 21, **caractérisée par le fait que** celle-ci se présente sous la forme d'une émulsion, d'un lait, d'une lotion, d'une crème, d'un polymère tensio-actif et émulsifiant, gélifiant et visqueux, d'une pommade, d'un shampooing, d'un savon, d'un gel, d'une poudre, d'un stick ou d'un crayon, d'une pulvérisation, d'une huile corporelle, d'un masque pour le visage ou d'un pansement pour une application transdermique.

24. Composition selon l'une des revendications 21 à 23, **caractérisée par le fait que** celle-ci contient des ingrédients utilisés de façon habituelle, lesquels sont choisis dans le groupe contenant : des lipides d'extraction et/ou des lipides de synthèse, des polymères gélifiants et visqueux, tensio-actifs et émulsifiants, des principes actifs solubles dans l'eau ou dans la matière grasse, des extraits de plantes, des extraits de tissus, des extraits marins, des agents de protection solaire, des anti-oxydants, des agents humectants et des agents de barrière et/ou des agents de revilatisation de la peau.

25. Composition selon l'une des revendications 21 à 24 comme agent dermopharmaceutiquement et/ou cosmétiquement actif, en particulier pour l'augmentation de la production de collagène et de fibronectine dans la peau humaine, pour l'accélération de la cicatrisation et de l'hydratation, comme agent de soins cutanés, en particulier pour prévenir la formation et l'aggravation des rides et contre toutes conséquences du vieillissement de la peau de type naturel ou accéléré (héliodermie, pollution).

26. Procédé cosmétique d'augmentation de la production de collagène et/ou de fibronectine dans la peau humaine et/ou d'accélération de l'hydratation et/ou de ralentissement ou de traitement du vieillissement cutané **caractérisé par le fait que** l'on applique sur la peau un composé selon l'une des revendications 1 à 17.
